# EUROPEAN PATENT SPECIFICATION

(11) **EP 1 744 756 B1**
(45) Date of publication and mention of the grant of the patent: **23.07.2008**
(21) Application number: 05736533.0
(22) Date of filing: 22.04.2005
(51) Int. Cl.: A61K 31/54, A61P 31/00

(54) **USE OF MELOXICAM FORMULATIONS IN VETERINARY MEDICINE**
VERWENDUNG VON MELOXICAM-FORMULIERUNGEN IN DER VETERINÄRMEDIZIN
UTILISATION DE FORMULATIONS A BASE DE MELOXICAME EN MEDECINE VETERINAIRE

(30) Priority: 29.04.2004 DE 102004021281
(43) Date of publication of application: 24.01.2007
(60) Divisional application: 08157816.3
(73) Proprietor: Boehringer Ingelheim Vetmedica GmbH, 55216 Ingelheim am Rhein (DE)
(72) Inventor: FRITON, Gabriele, 55411 Bingen (DE); LANG, Ingo, 55218 Ingelheim (DE)
(74) Representative: Hammann, Heinz
(86) International application number: PCT/EP2005/004344
(87) International publication number: WO 2005/105101

(56) References cited:
- WO-A-03/049733
- WO-A-20/05002542
- US-B1- 6 187 800
- HIRSCH ET AL: "Investigation on the efiicacy of meloxicam in sows with mastitis-metritis-agalactia syndrome" J.VET.PHARMACOL.THERAP., vol. 26, 2003, - 2003 pages 355-360, XP002338045

## Description

### FIELD OF THE INVENTION

The present invention is directed to the novel use of meloxicam formulations in veterinary medicine, especially for the treatment of mastitis in food-producing animals.

### BACKGROUND OF THE INVENTION

Mastitis is one of the most important diseases in dairy cattle and a serious cause of loss to the world's dairy industries. The US National Mastitis Council (NMC) estimates that annual losses to the dairy industry amount to US $ 1.8 to 2 billion or US $ 185 to 200 per cow. Losses due to discarded milk alone are thought to amount to US $ 1 billion. Up to 50% of all dairy cattle are thought to be affected by mastitis.

Mastitis is an inflammation of the mammary gland and results in significant losses to dairy industry due to production decrease or increased culling rates. Mastitis-causing pathogens can be divided into contagious pathogens that are associated with the udder (i.e. Staphylococcus aureus, Streptococcus agalactiae, and Streptococcus dysgalactiae) and environmental pathogens that are present in a cow's environment (coliform bacteria and streptococci other than Streptococcus agalactiae, and coagulase-negative staphylococci).

Infection may be clinically obvious with severe signs of illness or subclinically with no obvious signs. General clinical signs refer to increased rectal temperature (up to high fever) and severe deterioration in a cow's general state of health (no or reduced feed intake, impaired general condition). Local clinical signs include changes in macroscopic milk quality associated with typical inflammatory reactions of the affected quarter (red, swollen, warm and painful).

Both clinical and subclinical mastitis leads to reduced milk production. The extent of udder infection in a dairy cow is usually assessed through measuring the number of somatic cells present in milk.

Antibiotics are commonly used for treatment and prevention of infections of the udder and various products are available. Although new antibiotic presentations continue to be launched, animal health companies and the dairy industry are increasingly looking towards alternatives for treatment, metaphylaxis and prophylaxis of mastitis. Antibiotics are usually administered by intramammary injection and in severe cases of infection, they may be administered parenterally in addition. Intramammary preparations are supplied in disposable single-use syringes or tubes.

In order to reduce inflammation (anti-inflammatory) and body temperature (anti-pyretic) steroidal anti-inflammatory drugs (SAID) are well established in being used in combination with antibiotics. SAIDs can be administered systemically in combination with parenteral and/or intramammary antibiotic therapy. For intramammary treatment several products are available as combination products (antibiotic and SAID). However, most of these products failed the re-registration procedure, for example due to old data and the necessity to invest in full development for further product supply. The steroidal injectors will likely disappear in the future, although they have the biggest market size at the moment.

Recently, the value of NSAIDs (non-steroidal anti-inflammatory drugs) in mastitis therapy, i.e. in acute clinical mastitis cases, became of great importance, because NSAIDs have no immunosuppressive effect in comparison to SAIDs and they show proven efficacy in reduction of inflammation (anti-inflammatory), pain (analgesic), body temperature (anti-pyretic) and endotoxin associated signs. Currently there are several NSAIDs commercially available for cattle and licensed in several countries within the EU i.e. flunixin meglumine (Finadyne^{®} Injection, Schering-Plough Animal Health), ketoprofen (Ketofen^{®} 10%, Merial), meloxicam (Metacam^{®}, Boehringer Ingelheim) and tolfenamid acid (Tolfedine^{®}). All products are licensed for being administered parenterally.

First preliminary data are available for intramammary use of the NSAID flunixin meglumine (Fitzpatrick, J., Young F.J., Eckersall, D., Logue D.N., Knight C. J., Nolan, A., 1998, Recognizing and controlling pain and inflammation on mastitis Proceedings, British Mastitis Conference). Flunixin was infused intramammarily in a special prepared vehicle together with an antibiotic (amoxicillin/clavulanic acid) created by Schering-Plough Animal Health. However, the use of flunixin had no beneficial effect on pain perception i.e. the intramammary treatment of mastitis cases caused an adverse effect with increased responsiveness to pain. This could have been caused by irritation of the NSAID flunixin, when administered locally. These findings were also described in literature for local administration of NSAIDs (Löscher W., Ungemach F.R. und Kroker R., Pharmakotherapie bei Haus- und Nutztieren, 5. überarbeitete Auflage, aus: Lokale Therapie (Haut, Euter, Auge) Ungemach F.R., Kietzmann M. und Ehinger A.M., S. 445, 2002.

Currently there is no NSAID intramammary injector available on the market for the treatment of acute mastitis cases in lactating cows.

However, there exist prior art which is directed to the pharmaceutical use or the properties of meloxicam:

EP-A-0 002 482 shows, inter alia, the example of a 2 % injectable solution of meloxicam consisting of the meglumine salt of the active substance, sodium chloride and water.

EP-A-0 945 134 discloses the pH-dependent solubility characteristics of meloxicam and its salts, i.e. the sodium salt, the ammonium salt and the meglumine salt, in aqueous solution.

WO 99/59634 A1 describes an eye drop solution containing 0.5 % meloxicam.

Further, commercially available 0.5 % and 2 %meloxicam solution are used in small animals, cattle and pigs. The indications are inflammatory diseases as e.g. respiratory disease, lameness and mastitis.

Finally, WO 03/049733 describes a highly concentrated stable meloxicam solution for needleless injection containing from 35 to 100 mg/ml of dissolved meloxicam salt and one or more suitable additives for treating respiratory diseases and inflammation in mammals.

Therefore, it is an object of the present invention to provide a pharmaceutical composition for the treatment of inflammatory diseases, particularly mastitis, especially acute cases, which overcomes the above-mentioned problems related with the use of known NSAIDs. Furthermore, the inventive treatment should be possible as adjunctive therapy to antibiotics and/or as systemic treatment.

### DESCRIPTION OF THE INVENTION

Surprisingly, it has been found that suitable meloxicam formulations may be used for intramammary treatment of acute mastitis cases in order to reduce local clinical signs of inflammation in the udder and to relief pain.

Therefore, the present invention provides the use of a formulation containing meloxicam or a pharmacologically acceptable meloxicam salt of an organic or inorganic base, one or more vehicles and one or more suitable additives for preparing a veterinary medical composition for intramammary treatment of microbially induced diseases in mammals, particularly for preparing a veterinary medical composition for intramammary treatment of mastitis.

As already described, NSAIDs up to now known in prior art are not for intramammary use. The above-mentioned studies have shown that the local administration of NSAIDs, e.g. the intramammary infusion, causes an adverse effect with increased responsiveness to pain. Consequently, it could not be expected that one of the NSAIDs, i.e. meloxicam, may be used directly on the udder or may be applied into the udder without showing the negative effects provided by the other NSAIDs and resulting in excellent healing effects.

Actually, the use of the meloxicam formulation of the present invention leads to a decrease of the local inflammation symptoms when introduced through the teat canal in a quarter of the udder suffering from inflammatory disease such as mastitis. The meloxicam formulation used according to the invention exhibits a local anti-inflammatory effect, leading to a clinical improvement as well as a reduction of the concentration of the inflammatory mediators.

The meloxicam containing formulation has a good local tolerance. The intramammary administration leads to an effective concentration in the target tissue, which is achieved particularly quick.

According to a preferred embodiment the formulation according to the present invention contains meloxicam or meloxicam salt in a concentration of 0,1 to 50 mg/ml, preferably 0.5 to 30 mg/ml, more preferably 1 to 20 mg/ml, particularly preferably 2 to 15 mg/ml, especially preferably 3 to 8 mg/ml, especially 4 to 6 mg/ml. The most preferred concentration is 5 mg/ml.

The preferred quantity of meloxicam or meloxicam salt per udder quarter according to the present invention is a quantity of 1 to 100 mg, preferably 5 to 60 mg, more preferably 10 to 40 mg, particularly preferably 15 to 40 mg, especially preferably 20 to 40 mg, especially 25 to 35 mg. The most preferred quantity is 30 mg.

Therefore, the active substance is administered in a low quantity ranging from 1 to 50 mg. It is therefore possible to minimise the use of the pharmaceutical agent required for treating inflammatory diseases.

The meloxicam containing formulation used in the present invention may contain, in addition to meloxicam or a meloxicam salt, one or more vehicles and one or more additives. Depending from the vehicle the other additives are selected accordingly. The vehicle may be selected from water and/or oil to result in an aqueous or oily system; intermediate systems are also possible.

The term "aqueous or oily system" according to the present invention should be understood that the main part of the vehicle is derived from water or oil. The "vehicle" should be understood to be the medium or carrier which essentially disperses the active substance, i.e. the meloxicam, and the additives, such that the formulation is formed as a solution such as an aqueous solution, a hydrogel, an emulsion such as a microemulsion, an oil-in-water emulsion or a water-in-oil emulsion, or a suspension or the like.

In the frame of the present invention the expression "solution" should be understood to comprise dispersed systems as well as true solutions and intermediate states.

If an aqueous system is selected the meloxicam is used in the form of a salt. The meloxicam salt used according to the present invention may be the meglumine, sodium, potassium or ammonium salt, preferably may be mentioned the meloxicam meglumine salt.

Meglumine and meloxicam may be used in a molar ratio of from 9:8 to 12:8, preferably in a molar ratio of 11:8, but especially in a molar ratio of about 10:8.

In an aqueous medium it is advantageous if the additives are selected from the group consisting of solubilisers, preservatives, buffer substances for achieving the optimum pH range and optionally other additives. The system may for example optionally contain a suitable gelling agent and/or viscosity enhancer leading to a viscous aqueous solution or a hydrogel. Suitable systems can be sterile viscous aqueous solutions or hydrogels, sterile emulsions (e.g. oil-in-water), or sterile oily suspensions.

Further additives may be selected from the group consisting of citric acid, lecithin, gluconic acid, tartaric acid, phosphoric acid and EDTA or the salts thereof.

If an oily system is selected the additives may preferably be selected from one or more oils, one or more antioxidants and optionally one or more thickeners. It is a matter of course that also other additives may be present.

Suitable formulations are preparations for intramammary use (in accordance with the requirements of e.g. Ph. Eur.), containing the active substance meloxicam in a very low concentration of for example 0.1 to several percent, for example about 2 %, the additives being present in the formulation will be described hereinafter in detail.

The additives may be selected from the group consisting of buffers, solubilisers, gelling agents, viscosity enhancers, preservatives, oils, antioxidants, emulsifiers, foam forming agents, isotonic agents, a propellant gas and/or thickeners. However, even if the additives are discussed either under aqueous systems or under oily systems it is also possible that said additives are present in the other system, if required or desired.

### Preferred additives of the aqueous systems:

As solubilisers may be used any known solubiliser suitable in the veterinary medical sector, for example polyethylene glycols, polyoxyethylene-polyoxypropylene copolymers (e.g. poloxamer 188), glycofurol, arginine, lysine, castor oil, propyleneglycol, solketal, polysorbate, glycerol, sorbitol, mannitol, xylitol, polyvinylpyrrolidone, lecithin, cholesterol, 12-hydroxystearic acid-PEG660-ester, propyleneglycol monostearate, polyoxy-40-hydrogenated castor oil, polyoxyl-10-oleyl-ether, polyoxyl-20-ceto-stearylether and polyoxyl-40-stearate or a mixture of sorbitol, mannitol and xylitol, preferably polyethyleneglycols, polyoxyethylene-polyoxypropylene copolymers, glycofurol, polyvinylpyrrolidone, lecithin, cholesterol, 12-hydroxy-stearic acid-PEG660-esters, propyleneglycol monostearate, polyoxy-40-hydrogenated castor oil, polyoxyl-10-oleyl-ether, polyoxyl-20-cetostearylether and polyoxyl-40-stearate. Particularly preferred are polyethyleneglycols, glycofurol and polyoxyethylene-polyoxypropylene-copolymers, but especially polyethyleneglycols (e.g. Macrogol 300) and polyoxyethylene-polyoxypropylene copolymers (e.g. Poloxamer 188).

Any preservatives known for use in the pharmaceutical field may be used, for example, ethanol, benzoic acid and the sodium or potassium salts thereof, sorbic acid and the sodium or potassium salts thereof, chlorobutanol, benzyl alcohol, phenylethanol, phenylmercury nitrate, methyl-, ethyl-, propyl- or butyl-p-hydroxybenzoates, phenol, m-cresol, p-chloro-m-cresol or benzalkonium chloride. Particularly preferred are ethanol, benzoic acid and the sodium or potassium salts thereof, sorbic acid and the sodium or potassium salts thereof, chlorobutanol, benzylalcohol, phenylethanol and methyl, ethyl, propyl or butyl p-hydroxybenzoates, but preferably ethanol, benzoic acid and the sodium or potassium salts thereof, sorbic acid and the sodium or potassium salts thereof, but especially ethanol.

It may be advantageous if the formulation containing an aqueous medium according to the invention has a pH value in the alkaline range. Then, the pH value may be adjusted in the range from 8 to 10, preferably from 8.5 to 9, more preferably a pH from 8.7 to 8.9, particularly 8.8. However, also a pH value in the acidic range may be possible but an alkaline pH range is particularly preferred. In the more alkaline region the meloxicam containing formulation tends preferably to be a true aqueous solution whereas in the more acidic region it tends rather to be a suspension.

Therefore, the buffer system used to achieve a pH value of from 8 to 10 may be, for example, glycine, a mixture of glycine and HCl, a mixture of glycine and sodium hydroxide solution, and the sodium and potassium salts thereof, a mixture of potassium hydrogen phthalate and hydrochloric acid, a mixture of potassium hydrogen phthalate and sodium hydroxide solution or a mixture of glutamic acid and glutamate. Glycine, a mixture of glycine and HCl and a mixture of glycine/sodium hydroxide solution, especially glycine, are particularly preferred.

Suitable gelling agents are for example cellulose derivatives, e.g. hydroxyethyl cellulose.

Other suitable additives are for example citric acid, lecithin, gluconic acid, tartaric acid, phosphoric acid, isotonic agents such as sodium chloride and EDTA or the alkali metal salts thereof.

One preferably used formulation of the invention contains, in addition to the meglumine or sodium salt of the meloxicam, polyethyleneglycols, glycofurol and/or polyoxyethylene-polyoxypropylene copolymers, but particularly polyethyleneglycols (e.g. Macrogol 300) and/or polyoxyethylene-polyoxypropylene copolymers (e.g. Poloxamer 188) as solubiliser, ethanol, benzoic acid and the sodium or potassium salts thereof or sorbic acid and the sodium or potassium salts thereof, but particularly ethanol, as preservative, and glycine, a mixture of glycine/HCl or a mixture of glycine/sodium hydroxide solution, but preferably glycine, as buffer and optional disodium EDTA as an additional additive.

### Preferred additives of the oily systems:

Suitable oily components are any of the active substances known from the prior art for the preparation of pharmaceuticals, such as, for example, vegetable oils, in particular, e.g. cotton seed oil, peanut oil, maize oil, rapeseed oil, sesame oil and soya oil, or triglycerides of moderate chain length, e.g. fractionated coconut oil, or isopropylmyristate, -palmitate or mineral oils or ethyloleate or mixtures thereof. Preferred oils may be selected from vegetable oils, such as corn seed oil, sesame oil, and peanut oil.

The antioxidants used may be any of the antioxidants known from the prior art, preferably sesamol, alpha-tocopherol (vitamin E), butylhydroxytoluene (BHT) or butylhydroxyanisole (BELA).

The use of thickeners like e.g. aluminium monosterarate, hydrogenated castor oil, carboxymethyl cellulose or salts thereof can be suitable as well.

Other additives which may be present are emulsifiers, foam forming agents and propellant gases.

The preferred emulsifiers used, apart from the emulsifiers known from the prior art, include polyoxyethylene derivatives of castor oil or polyoxyethylene alkylethers.

The foam-forming agents used may be any of those which are permitted under the drug licensing laws and known from the prior art, preferably polyoxyethylene sorbitanesters of various fatty acids (polysorbates).

Suitable propellant gases are all those which are licensed for use in the medical field and those which are known from the prior art, e.g. CO₂, N₂O, N₂, propane/butane mixtures, isobutane, chloropentafluoroethane (CCIF₂-CF₃), octafluorocyclobutane (C₄F₈).

Some formulations by way of example follow the experimental section.

The formulation according to the invention, that is the aqueous or oily system, may have a usual shelf-life after opening of several weeks or more at ambient temperature.

The formulation used according to the invention is suitable for treating microbially induced diseases, particularly mastitis, also acute cases. It is suitable for treating mammals, particularly working animals or farm animals. The treatment may be given in conjunction with antibiotic therapy administered systematically and/or locally.

The formulation according to the invention may be prepared using the methods of preparing formulations known from the literature. For example, the appropriate additives may be added to a meloxicam/meloxicam salt preparation.

The meloxicam containing formulation of the invention may be administered in the form of creams, ointments, lotions, water-in-oil or oil-in-water emulsions, aerosol foams or injector formulations. The preparation of pharmaceutical forms of this kind is well-known per se from the prior art.

Preferably use is made of an injector formulation. An injector comprises means which allows to inject the formulation intramammary, i.e. through the teat canal into the udder, with the formulation being present in a casing, reservoir, phiole, syringe or tube or the like, which may be disposable and provided for a single-use, containing a delivery system such as a suitable opening, channel or a blunt needle. This form of intramammary application may achieve a good distribution in the target organ together with an increase in the activity.

### The advantages of the present invention are manifold:

The use of the formulation according to the invention overcomes the problem arising from the prior art by providing a preparation of the active substance meloxicam/meloxicam salt which may be intramammary administered for the treatment of inflammatory diseases, particularly mastitis.

Although the udder tissue is a highly sensitive tissue the use of the meloxicam formulation of the present invention has a good local tolerance. The other known NSAIDs which are can only be parenterally administered are not suitable to be applied intramammary because they would cause tissue irritation.

On the contrary, the use of the meloxicam formulation of the invention introduced in a quarter of the udder suffering from inflammatory disease, e.g. mastitis, leads to a decrease of the local inflammation symptoms. The meloxicam formulation exhibits a local anti-inflammatory effect, leading to a clinical improvement as well as a reduction of the concentration of the inflammatory mediators. Furthermore, the intramammary administration leads to an effective concentration in the target tissue, which is achieved very quickly.

The experimental findings described hereinafter provide clear evidence of promising treatment of mastitis in mammals by the use of a low quantity of meloxicam in the present invention.

The invention described will now be illustrated by the Examples which follow various other embodiments and will become apparent to the skilled person from the present specification. However, it is expressly pointed out that the Examples and description are intended solely as an illustration and should not be regarded as restricting the invention.

### Examples

In the following Examples 1 to 3 formulations according to the invention were prepared for intramammary use (in accordance with the requirements of Ph. Eur.) containing meloxicam or meloxicam salt in an aqueous or oily system. The formulations are listed in the following tables 1 to 3.

The further Examples 4 and 5 describe studies which have been conducted to show the local tissue tolerance and efficacy of the formulations of the present invention. **Example 1:**

Formulation 1 of the invention was prepared in form of an injector solution.

**Table 1**

| **ingredient** | **g/100 ml** |
|---|---|
| Meloxicam | 0.500 |
| Meglumine | 0.3125 |
| Glycofurol | 10.000 |
| Poloxamer 188 | 5.000 |
| Ethanol | 15.000 |
| Sodium chloride | 0.600 |
| Glycine | 0.500 |
| Sodium hydroxide | q.s. to give pH 8.7 |
| Water for injection | ad 100 ml |

### Example 2:

Formulation 2 of the invention was prepared in form of an injector solution.

**Table 2**

| **ingredient** | **g/100 ml** |
|---|---|
| Meloxicam | 0.500 |
| Meglumine | 0.3125 |
| Glycofurol | 10.000 |
| Poloxamer 188 | 5.000 |
| Carboxymethylcellulose Sodium | 5.000 |
| Ethanol | 15.000 |
| Sodium chloride | 0.600 |
| Glycine | 0.500 |
| Sodium hydroxide | q.s. to give pH 8.7 |
| Water for injection | ad 100 ml |

### Example 3:

Formulation 3 of the invention was prepared in form of an oily suspension.

**Table 3**

| **ingredient** | **g/100 ml** |
|---|---|
| Meloxicam | 2.000 |
| Aluminium monostearate | 2.000 |
| Alpha Tocopherol | 0.050 |
| Sesame Oil | ad 100 ml |

### Example 4:

### - Local tissue tolerance study -

A study was conducted in 1983 investigating the tolerance of meloxicam in the mammary gland of lactating cows (Ziv G., 1983, Udder irritation studies with compound UH-AC 62 XX in cows, Internal Report of Boehringer Ingelheim GmbH, not published up to now). Overall, 12 cows were included into the study: 8 lactating cows with normal udders i.e. no pathogens were isolated, somatic cell count (SCC) was less than 500 000 cells/ml milk and 4 cows with at least one quarter of the udder secreting milk with more than 750 000 cells/ml milk.

Meloxicam was dissolved in peanut oil and a volume of 10 ml, containing 10 mg meloxicam, was infused into the udder. The two left side quarters were treated with meloxicam, whereas in the right side quarters 10 ml peanut oil was infused (control). In 8 cows (4 cows with normal udder secretion and 4 cows with increased somatic cell counts) treatment was administered once, in another 4 cows (normal udder secretion) treatment was repeated after 24 hours. There was a transient, mild rise in SCC after single and repeated treatments, but somatic cell counts never exceeded 500 000 SCC/ml in the normal quarters and decreased to pre-treatment levels on days 2 or 3 after treatment. In general, treatment with meloxicam resulted in only slightly higher SCC compared to the control quarters. In the transient inflamed quarters, SCC remains high after infusion of meloxicam or peanut oil. An increase in SCC would indicate a poor local tolerance.

Therefore, meloxicam caused no tissue irritation in normal and transient inflamed quarters when administered intramammarily at a dose of 10 mg meloxicam per quarter.

### Example 5:

### - Efficacy and dose-determination -

### Study design and methods

A blinded in-vivo study with a parallel group design was conducted in 2003. The study was conducted according to Good Laboratory Practices.

Clinical mastitis was experimentally induced in 20 lactating cows by intramammary infusion of E. coli endotoxin 026:B6 lipopolysaccharide in one hind quarter. 2 hours after endotoxin-challenge, the challenged udder quarters were treated with local administration of meloxicam in an experimental formulation. The cows were allocated to 4 treatment groups, each with 5 cows:
untreated, 10 mg meloxicam, 30 mg meloxicam, 50 mg meloxicam per endotoxin-challenged udder quarter.

The animals were clinically examined before endotoxin-challenge and at 2 h (before treatment), 4 h, 6 h, 8 h, 10 h, 24 h, 34 h, 48 h, 72 h, 7 days, and 14 days after endotoxin-challenge.

The size of the challenged udder quarter was documented using the following score:
0 Normal
1 Slight increase up to 20 %
2 20-50 % increase
3 More than 50 % increase

Ruminal contractions during a 2 minutes period were examined by auscultation in the left paralumbar fossa. In painful conditions of any kind, the frequency of ruminal contractions in ruminants tends to decrease.

Milk samples for analysis of thromboxane B₂ (TBX) - a relevant mediator of inflammation - were taken from the endotoxin-challenged quarter before endotoxin challenge and at 2 h (before treatment), 4 h, 6 h, 8 h, 10 h, and 24 h after endotoxin-challenge.

Milk somatic cell count (SCC) was measured before endotoxin-challenge and at 10 h, 24 h, 34 h, 48 h, 72 h, 7 days, and 14 days after challenge.

### Results

2 h after endotoxin-challenge, the cows developed systemic and local symptoms of mastitis. The cows showed disturbed general demeanour, increased respiratory rate, increased rectal temperature; and decreased frequency of ruminal contractions. The local symptoms were swelling, pain and altered secretion in the challenged udder quarters. These symptoms were most severe 4-6 hours after the endotoxin challenge. Thereafter, they diminished during the 24 hours after endotoxin-challenge.

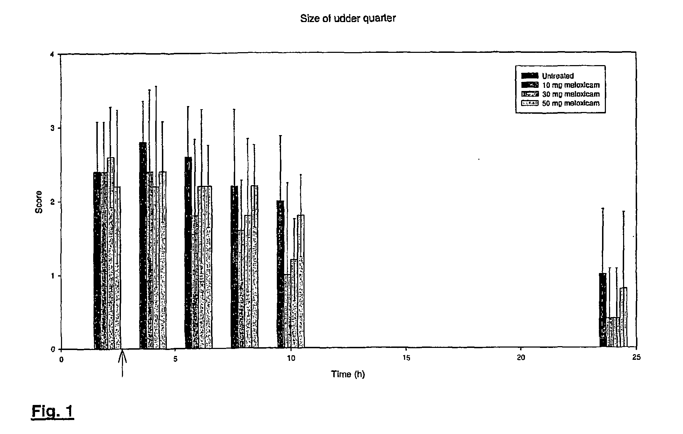

The size of the endotoxin-challenged udder quarters during the 24 h after challenge is displayed in Fig. 1.

Note: In all graphs, means ± 95 % confidence intervals by treatment group are displayed. The arrows indicate treatment with meloxicam - immediately after the examination at 2 h.

Before endotoxin-challenge - at 0 h -, all udder quarters had score 0. At 2 h - before treatment - the udder quarters showed relevant increases in size. There was a clear tendency that the untreated udder quarters were larger during the 24 h after challenge. To illustrate the relevance of this difference, the mean scores of the untreated and 30 mg meloxicam group at 10 h are compared as an example. Mean ± standard deviation (S.D.) in the untreated group was 2.2 ± 0.84, whereas in the 30 mg meloxicam group it was 1.2 ± 0.44. Thus the standardised difference - difference in means divided by S.D. - was 1.2 when calculated with the larger S.D. (untreated group) as a worst case scenario. According to Cohen's evaluation of standardised differences, a standardised difference > 0.8 is considered as large.

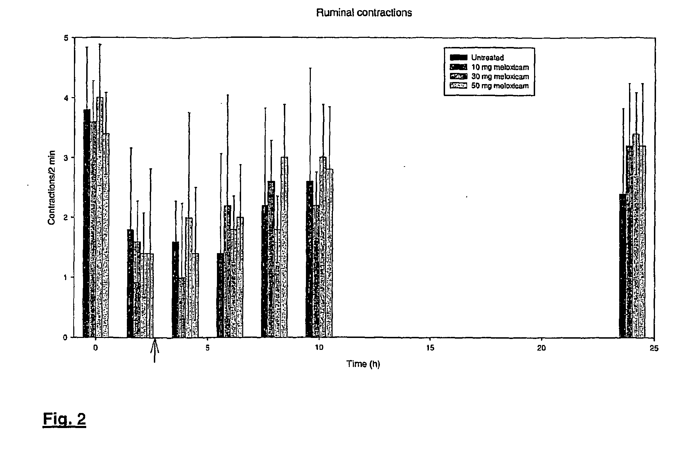

The frequency of ruminal contractions decreased in all treatment groups after challenge with a minimum between 2 and 6 h (see Fig. 2: Ruminal concentrations in 2 min.). The frequency of ruminal contractions tended to increase earlier in the meloxicam-treated cows. At 24 h, mean ± S.D. in the untreated group was 2.4 ± 1.14, in the 30 mg meloxicam group it was 3.4 ± 0.55.

Thus the standardised difference - calculated with the larger S.D. (untreated group) - was 0.9. This indicates a large difference according to Cohen (see above).

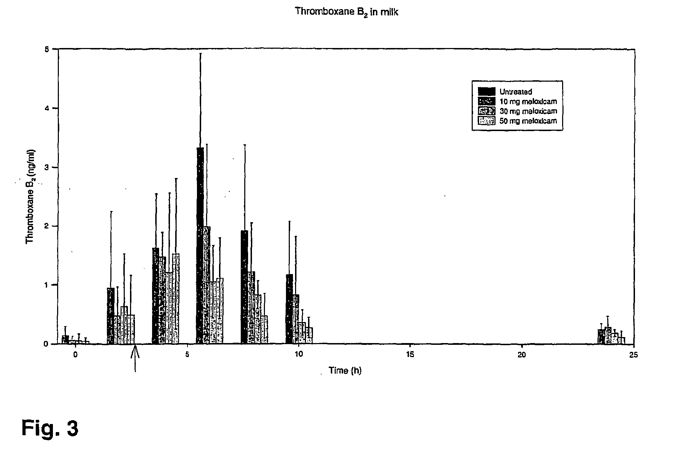

TBX-concentrations in the milk of the challenged quarters were low before challenge. After challenge, a large rise in TBX-concentration was observed (see Fig. 3: Thromboxane B₂ concentrations in the milk of the endotoxin-challenged udder quarter). The lower limit of quantification was 0.3 ng/ml, the upper limit 4 ng/ml. Values beyond these limits were set to 0.3 and 4 ng/ml, respectively. TBX-concentrations in the treated quarters reached lower peak concentrations than the untreated quarters. Moreover, there was a tendency for a faster decrease in TBX-concentration. The differences between untreated and meloxicam treated quarters are clearly relevant. At 6 h after challenge - the time with the peak TBX-concentration - the 95 % confidence intervals of the untreated and the 30 mg meloxicam treated quarters do not overlap, indicating a statistically significant difference.

Clinical examinations and SCC revealed no signs of poor local tolerance of the meloxicam treatment in the two weeks after treatment.

### Conclusion

Treatment with intramammary meloxicam in a dose range of 10-50 mg per affected udder quarter reduced local inflammation and pain in endotoxin-induced bovine mastitis. Local and systemic tolerance were good.

## Claims

1. Use of a formulation containing meloxicam or a pharmacologically acceptable meloxicam salt of an organic or inorganic base, one or more vehicles and one or more suitable additives for preparing a veterinary medical composition for intramammary treatment of inflammatory diseases in mammals.

2. Use of a formulation according to claim 1 for preparing a veterinary medical composition for intramammary treatment of mastitis.

3. Use of a formulation according to claim 1 or 2, **characterised in that** the quantity of meloxicam or meloxicam salt being from 1 to 50 mg per application.

4. Use of a formulation according to claims 1 to 3, **characterised in that** the vehicle is selected from water and/or oil.

5. Use of a formulation according to claim 4 **characterised in that** it is in form of an aqueous solution, hydrogel, emulsion, or suspension.

6. Use of a formulation according to one of the preceding claims 1 to 5, **characterised in that** the meloxicam salt is the sodium, potassium, ammonium or meglumine salt.

7. Use of a formulation according to one of the preceding claims 1 to 6, **characterised in that** it contains one or more additives selected from the group consisting of buffers, solubilisers, gelling agents, viscosity enhancers, preservatives, oils, antioxidants, emulsifiers, foam forming agents, isotonic agents, a propellant gas and/or thickeners.

8. Use of a formulation according to one of the preceding claims 1 to 7, **characterised in that** the aqueous formulation has a pH value from 8.0 to 10.

9. Use of a formulation according to one of the preceding claims 1 to 8, **characterized in that** it contains or essentially consists of meloxicam, meglumine, a polyethyleneglycol, a polyoxyethylene-polyoxypropylene copolymer, ethanol, glycine, water and optionally sodium hydroxide, hydrochloric acid and/or disodium EDTA.

10. Use of a formulation according to one of the preceding claims 1 to 9, **characterized in that** it contains or essentially consists of meloxicam, one or more oils, one or more antioxidants and optionally one or more thickeners.

## Patentansprüche

1. Verwendung einer Formulierung, enthaltend Meloxicam oder ein pharmazeutisch akzeptables Meloxicamsalz einer organischen oder anorganischen Base, ein oder mehrere Vehikel bzw. Trägermittel und ein oder mehrere geeignete Zusatzstoffe, zur Herstellung einer veterinärmedizinischen Zusammensetzung zur intramammären Behandlung von inflammatorischen Erkrankungen von Säugetieren.

2. Verwendung einer Formulierung nach Anspruch 1 zur Herstellung einer veterinärmedizinischen Zusammensetzung zur intramammären Behandlung von Mastitis.

3. Verwendung einer Formulierung nach Anspruch 1 oder 2, **dadurch gekennzeichnet, dass** die Menge an Meloxicam oder Meloxicamsalz von 1 bis 50 mg pro Anwendung reicht.

4. Verwendung einer Formulierung nach den Ansprüchen 1 bis 3, **dadurch gekennzeichnet, dass** das Vehikel ausgewählt ist aus Wasser und/oder Öl.

5. Verwendung einer Formulierung nach Anspruch 4, **dadurch gekennzeichnet, dass** sie in Form einer wässerigen Lösung, eines Hydrogels, einer Emulsion oder einer Suspension vorliegt.

6. Verwendung einer Formulierung nach irgendeinem der vorhergehenden Ansprüche 1 bis 5, **dadurch gekennzeichnet, dass** das Meloxicamsalz das Natrium-, Kalium-, Ammonium- oder Megluminsalz darstellt.

7. Verwendung einer Formulierung nach irgendeinem der vorhergehenden Ansprüche 1 bis 6, **dadurch gekennzeichnet, dass** sie einen oder mehrere Hilfsstoffe enthält, ausgewählt aus der Gruppe, bestehend aus Puffern, Lösungshilfsmitteln, Gelbildnern, Viskositätsverbesserern, Konservierungsmitteln, Ölen, Antioxidantien, Emulgatoren, Schaumbildnern, isotonischen Mitteln, einem Treibgas und/oder Verdickern.

8. Verwendung einer Formulierung nach irgendeinem der vorhergehenden Ansprüche 1 bis 7, **dadurch gekennzeichnet, dass** die wässerige Formulierung einen pH-Wert von 8,0 bis 10 aufweist.

9. Verwendung einer Formulierung nach irgendeinem der vorhergehenden Ansprüche 1 bis 8, **dadurch gekennzeichnet, dass** sie enthält oder im Wesentlichen besteht aus Meloxicam, Meglumin, einem Polyethylenglykol, einem Polyoxyethylen-Polyoxypropylen-Copolymer, Ethanol, Glycin, Wasser und gegebenenfalls Natriumhydroxid, Salzsäure und/oder Dinatrium-EDTA.

10. Verwendung einer Formulierung nach irgendeinem der vorhergehenden Ansprüche 1 bis 9, **dadurch gekennzeichnet, dass** sie enthält oder im Wesentlichen besteht aus Meloxicam, einem oder mehreren Ölen, einem oder mehreren Antioxidantien und gegebenenfalls einem oder mehreren Verdickern.

## Revendications

1. Utilisation d'une formulation contenant du méloxicam ou un sel pharmacologiquement acceptable du méloxicam et d'une base organique ou inorganique, un ou plusieurs véhicules et un ou plusieurs additifs appropriés pour préparer une composition médicale vétérinaire pour le traitement intramammaire des maladies inflammatoires chez les mammifères.

2. Utilisation d'une formulation selon la revendication 1 pour préparer une composition médicale vétérinaire pour le traitement intramammaire de la mastite.

3. Utilisation d'une formulation selon la revendication 1 ou 2 **caractérisée en ce que** la quantité de méloxicam ou de sel de méloxicam est de 1 à 50 mg par application.

4. Utilisation d'une formulation selon les revendications 1 à 3 **caractérisée en ce que** le véhicule est choisi parmi l'eau et/ou une huile.

5. Utilisation d'une formulation selon la revendication 4 **caractérisée en ce qu'**elle est sous forme d'une solution aqueuse, d'un hydrogel, d'une émulsion ou d'une suspension.

6. Utilisation d'une formulation selon l'une quelconque des revendications 1 à 5 précédentes **caractérisée en ce que** le sel de méloxicam est le sel de sodium, de potassium, d'ammonium ou de méglumine.

7. Utilisation d'une formulation selon l'une des revendications 1 à 6 précédentes **caractérisée en ce qu'**elle contient un ou plusieurs additifs choisis dans le groupe consistant en les tampons, les solubilisants, les agents gélifiants, les agents augmentant la viscosité, les conservateurs, les huiles, les antioxydants, les émulsifiants, les agents moussants, les agents d'isotonie, un gaz propulseur et/ou les épaississants.

8. Utilisation d'une formulation selon l'une des revendications 1 à 7 précédentes **caractérisée en ce que** la formulation aqueuse a un pH de 8,0 à 10.

9. Utilisation d'une formulation selon l'une des revendications 1 à 8 précédentes **caractérisée en ce qu'**elle contient ou consiste essentiellement en du méloxicam, de la méglumine, un polyéthylèneglycol, un copolymère polyoxyéthylène-polyoxypropylène, de l'éthanol, de la glycine, de l'eau et éventuellement de l'hydroxyde de sodium, de l'acide chlorhydrique et/ou de l'EDTA de disodium.

10. Utilisation d'une formulation selon l'une des revendications 1 à 9 précédentes **caractérisée en ce qu'**elle contient ou consiste essentiellement en du méloxicam, une ou plusieurs huiles, un ou plusieurs antioxydants et éventuellement un ou plusieurs épaississants.
